(19) Europäisches Patentamt
European Patent Office
Office européen des brevets

(11) Veröffentlichungsnummer: **0 144 750**
**B1**

(12) # EUROPÄISCHE PATENTSCHRIFT

(45) Veröffentlichungstag der Patentschrift: **08.06.88**

(51) Int. Cl.⁴: **C 07 H 19/06**, C 07 K 9/00, C 07 K 5/08, C 12 P 19/38

(21) Anmeldenummer: **84113276.4**

(22) Anmeldetag: **05.11.84**

(54) **Neue Antibiotika, Verfahren zu ihrer Herstellung, ihre Verwendung als Arzneimittel und Zwischenprodukte zu ihrer Herstellung.**

(30) Priorität: **17.11.83 DE 3341571**

(43) Veröffentlichungstag der Anmeldung:
**19.06.85 Patentblatt 85/25**

(45) Bekanntmachung des Hinweises auf die Patenterteilung:
**08.06.88 Patentblatt 88/23**

(84) Benannte Vertragsstaaten:
**AT BE CH DE FR GB IT LI NL SE**

(56) Entgegenhaltungen:
**EP-A-0 057 349**
**EP-A-0 057 812**
**EP-A-0 069 882**
**EP-A-0 105 393**

**LIEBIGS ANNALEN DER CHEMIE, 10. August 1984, Seiten 1399-1407,Verlag Chemie GmbH, Weinheim, DE;G. BENZ: "Enzymatische Spaltung der Desferriform der Albomycine delta 1, delta 2**
**LIEBIGS ANNALEN DER CHEMIE, 9. Dezember 1983, Seiten,1434-1440 Verlag Chemie GmbH, Weinheim, DE; G. BENZ u.a.:"Isolierung und Totalsynthese von (N5-Acetyl-N5-hydroxy-L-ornithyl)-(N5-acetyl-N5-hydroxy -L-ornithyl)-N5-acetyl-N5-hydroxy-L-ornithin"**

(73) Patentinhaber: **BAYER AG, Konzernverwaltung RP Patentabteilung, D-5090 Leverkusen 1 Bayerwerk (DE)**

(72) Erfinder: **Benz, Günter, Dr., 1818 Fordem Avenue 6, Madison/WI 53704 (US)**
Erfinder: **Metzger, Karl Georg, Dr., Pahlkestrasse 75, D-5600 Wuppertal 1 (DE)**
Erfinder: **Pfitzner, Jörg, Dr., Claudiusweg 3, D-5600 Wuppertal 1 (DE)**
Erfinder: **Schmidt, Delf, Dr., Am Eckbusch 55b, D-5600 Wuppertal 1 (DE)**
Erfinder: **Zeiler, Hans- Joachim, Dr., Elsbeekerstrasse 46, D-5620 Velbert 15 (DE)**

**EP 0 144 750 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die Erfindung betrifft neue Verbindungen der allgemeinen Formel 1

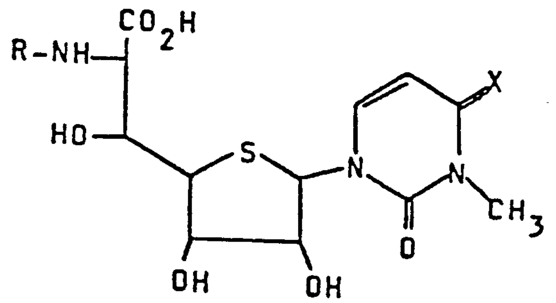

(1)

in der
X 0 oder N-CO-$NH_2$ und
R den Rest einer Aminosäure (außer Serin), einen Oligopeptidrest (außer HyoHyoHyoSer und seiner Derivate an der freien Aminogruppe) und einen Rest aus der Gruppe HyoSer, HyoHyoSer, HyoHyoHyoAla, HyoHyoHyoThr, Hyo, HyoHyo oder HyoHyoHyo darstellt, wobei Hyo die Bedeutung $N^5$-Acetyl-$N^5$-hydroxy-L-ornithin hat.

Die Beschränkungen in der Definition des Restes R beziehen sich auf die nicht vorveröffentlichte EP-0 105 393-A1, die als Stand der Technik unter Art. 54(3) EPÜ fällt.

R in der Bedeutung Aminosäure stellt bevorzugt den Rest einer natürlich vorkommenden Aminosäre dar, nämlich Glycin, Alanin, Valin, Leucin, Isoleucin, Phenylalanin, Tyrosin, Prolin, Hydroxyprolin, Threonin, Cystein, Cystin, Methionin, Tryptophan, Asparaginsäure, Asparagin, Glutaminsäure, Glutamin, Hydroxyglutaminsäure, Arginin, Lysin, Histidin oder einen Oligopeptidrest aus diesen Aminosäuren. Ganz besonders bevorzugt sind Alanin und Threonin.

Die erfindungsgemäßen Verbindungen können wie folgt hergestellt werden.

Man geht aus von einer Verbindung der Formel 2

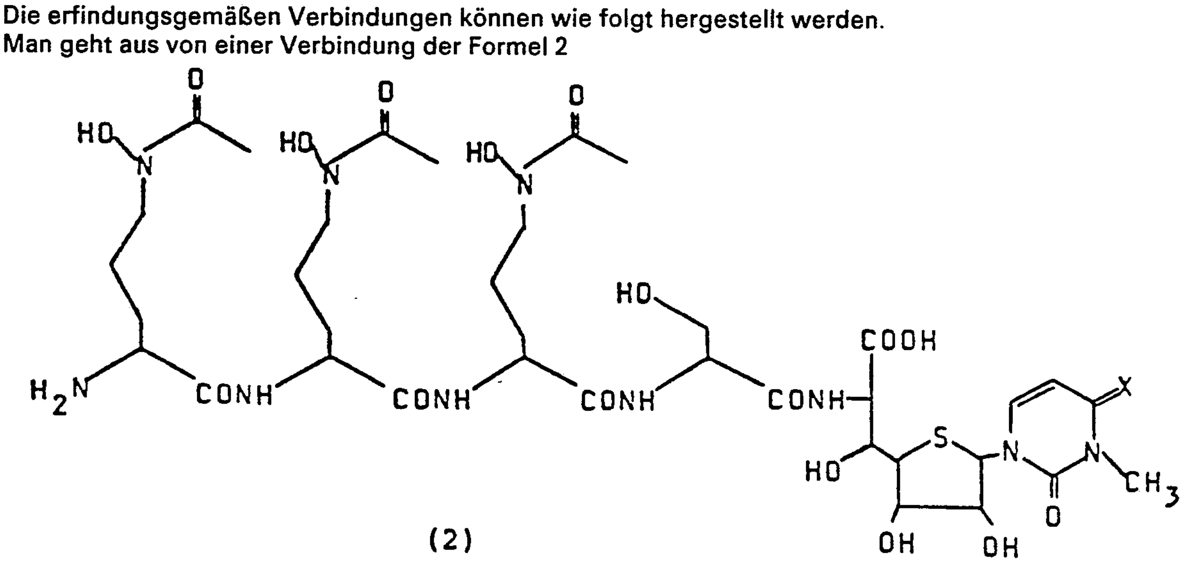

(2)

in der X die Bedeutung O oder N-CO-$NH_2$ hat. Diese Verbindungen und ihre Herstellung sind beschrieben in den deutschen Offenlegungsschriften 3 102 136 und 3 102 137 (entsprechend EP-A-57 349 und 57 812 sowie US Serial No. 340.418 und 340.449). Wenn man eine Verbindung der allgemeinen Formel (2) mit einem geeigneten Enzym spaltet, so erhält man - neben anderen Spaltprodukten - eine Verbindung der allgemeinen Formel 3

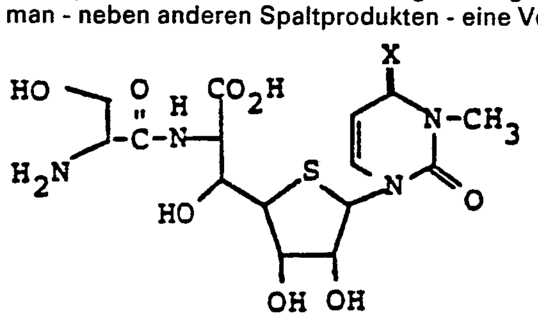

(3)

in der X = O oder N-CO-$NH_2$ ist.

Hierfür kommen vor allem Peptidhydrolasen der Gruppen EC 3.4.11 (α-Amino-acyl-peptidhydrolasen) und EC 3.4.21-23 (Proteasen) in Frage. Besonders zu nennen sind α-Chymotrypsin, Subtilisin, Aminoacylase I, β-

Lactamase aus E.coli T7, Proteinase K und Pronase K.

Bei der Inkubation von (2) mit mikrosomaler Leucinaminopeptidase werden die serinfreien Nucleoside (4) erhalten.

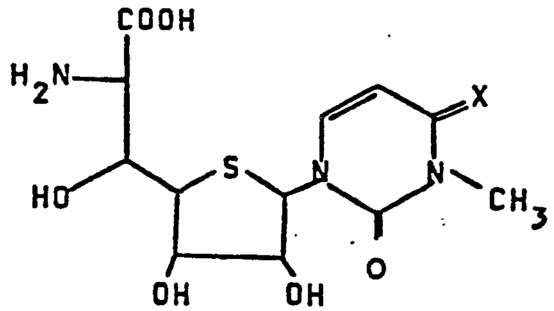

(4)

in der X die obengenannte Bedeutung hat.

Die Verbindungen (3) und (4) sind wertvolle Zwischenprodukte zur Herstellung der Verbindungen der allgemeinen Formel (1). Verbindung (4) gehört zur Erfindung.

Das bei der Spaltung von (2) nach (4) bevorzugt verwendete Enzym ist die mikrosomale Leucinaminopeptidase (Hersteller: Sigma; L 5006) mit einer spezifischen Aktivität von 10-20 U/mg (Leucinamid als Substrat). (Hierbei ist U die Einheit, die 1 µMol L-Leucinamid pro Minute bei pH 8,5 und 25°C zu Leucin und $NH_3$ hydrolysiert).

Die Spaltung wird vorgenommen in wäßrigem Medium bei einem pH-Wert von 6,6 bis 7,8, vorzugsweise bei etwa 7,2. Die Reaktionstemperatur liegt im allgemeinen bei 20 bis 40°C, vorzugsweise bei 37°C. Die Reaktionsdauer beträgt bei den zuvorgenannten Reaktionsbedingungen nicht unter 2 Tage, vorzugsweise etwa 6 bis 8 Tage.

Beim Einsatz der mikrosomalen Leucinaminopeptidase als Enzym werden - neben (4) - auch $N^5$-Acetyl-$N^5$-hydroxy-ornithin (5) und Serin (6) erhalten.

Im Anschluß an die Reaktion wird die jeweilige erfindungsgemäße Verbindung von etwa vorhandenen anderen Spaltprodukten abgetrennt, d. h. gereinigt.

Die Reinigung erfolgt dabei vorzugsweise durch Chromatographie. Vorzugsweise wird sie an Celluloseionenaustauschern (e. g. Sephadex® SP 25 H⊕, Natriumchloridgradient als Laufmittel), Adsorberharzen und an Kieselgel durchgeführt.

Bei dem erfindungsgemäßen ersten Verfahrensschritt der enzymatischen Spaltung muß es als überraschend angesehen werden, daß mit der mikrosomalen Leucinaminopeptidase die Spaltung bis zum Nucleosid (4) gelingt, da das Nucleosid (4) keine typische Aminosäure darstellt.

Ein neues eisenkomplexierendes Tripeptid [$N^5$-Acetyl-$N^5$-hydroxy-L-ornithyl]-[$N^5$-acetyl-$N^5$-hydroxy-L-ornithyl]-$N^5$-acetyl-$N^5$-hydroxy-L-ornithin (7) kann aus dem Kulturfiltrat einer Fermentation mit Streptomyces spec. WS 116 (DSM 1692)*) nach Adsorption an Lewatit® OC 1031. Chromatographie an seinem $Fe^{3+}$ beladenen Kationenaustauscher. Entsalzung an Lewatit® OC 1031, Chromatographie an einem stark- und nachfolgend einem schwachsauren Kationenaustauscher erhalten werden.

Das gleiche Tripeptid (7) wird bei der enzymatischen Spaltung der Verbindung (2) mit Endopeptidasen zum serinenthaltenden Nucleosid (3) gefunden. Hierbei sind Proteinase K und Subtilisin bevorzugt.

*) Der Stamm WS 116 wurde am 7. Dezember 1979 bei der Deutschen Sammlung von Mikroorganismen (DSM) unter dem Aktenzeichen DSM 1692 hinterlegt.

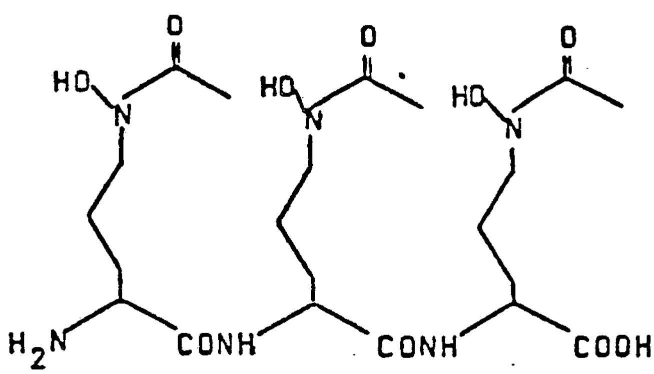

(7)

Das intakte Tripeptid (HyoHyoHyo) (7) kann als solches aus dem Reaktionsmedium isoliert werden. Es befindet sich als Partialstruktur in einem Teil der Verbindung (1) und gehört ebenfalls zur Erfindung.

Ausgehend von dem Serinnucleosid (3) wird das serinfreie Nucleosid (4) wiederum nur mit der mikrosomalen Leucinaminopeptidase erhalten. Die Aufarbeitung erfolgt wie oben angegeben, vorzugsweise durch Chromatographie.

Bei der weiteren Umsetzung von (3) und (4) zu (1) sind folgende Maßnahmen bevorzugt.

Die eingesetzten Aminosäuren und Peptide sind N-blockiert. Bevorzugte Schutzgruppen sind die BOC (t-Butyloxycarbonyl)- und die Z (Benzyloxycarbonyl)-Gruppe. Die nach üblichen peptidchemischen Verfahren im basischen Medium eingeführt werden. Die Hydroxyfunktion der Hydroxamsäure in Derivaten von $N^5$-Acetyl-$N^5$-hydroxy-ornithin (5) ist benzylgeschützt. Die Peptidverknüpfung erfolgt nach der Mixed-Anhydrid Methode. Die Aktivierung der geschützten Aminosäure bzw. des Peptids erfolgt in THF bei etwa -20°C, vorzugsweise mit Chlorameisensäureisobutylester/N-Methylmorpholin. Die Kupplung mit den Nucleosiden (3) und (4) wird dann in einem wäßrig organischen Medium, vorzugsweise in Wasser/THF-Gemischen vorgenommen. Alternativ können auch Aktivester (Hydroxysuccinimidester) N-blockierter Aminosäuren bzw. Peptide in die Kupplungsreaktion eingesetzt werden. Die Deblockierung erfolgt durch Hydrogenolyse über Pd (Z- und Benzylschutzgruppe) oder mit Trifluoressigsäure/Methylenchlorid 1 : 1 (BOC-Schutzgruppe).

Die so erhaltenen Verbindungen der Formel (1) werden an Sephadex® SP 25 H⊕ (Entwicklung mit einem Kochsalzgradienten) chromatographiert und an Lewatit® OC 1031 entsalzt.

Die folgende Tabelle gibt besonders bevorzugte Verbindungen der Formel (1) wieder.

**Tabelle 1**

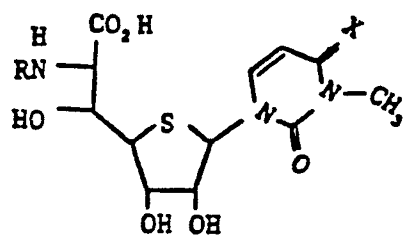

| Verbindung | X=N-CO-$NH_2$ R= | Beispiel | X=O R= |
|---|---|---|---|
| 8 | Ala | 21 | Ala |
| 9 | HyoHyoHyoAla | 22 | Hyo |
| 10 | His | 23 | HyoHyo |
| 11 | Hyo | 24 | HyoHyoHyo |
| 12 | HyoHyo | | |
| 13 | HyoHyoHyo | 25 | AlaSer |
| 14 | AlaSer | 26 | HisSer |
| 15 | HisSer | 27 | HyoSer |
| 16 | HyoSer | 28 | HyoHyoSer |
| 17 | HyoHyoSer | 29 | ThrSer |
| 18 | ThrSer | 30 | Thr |
| 19 | Thr | 31 | HyoHyoHyoThr |
| 20 | HyoHyoHyoThr | | |

Die erfindungsgemäßen Verbindungen (1) weisen antimikrobielle Wirksamkeit auf, die durch nachfolgende in vitro-Versuche demonstriert wird.

In vitro wird aufgrund der Hemmhofdurchmesser Wirksamkeit gegen die nachfolgend aufgeführten Keime gefunden.

**Tabelle 2**

| Verbindung | Lochtest | Hemmhofdurchmesser E.coli T 7 (ug/ml) 1000 | 100 | 10 | E.coli 14 (ug/ml) 1000 | 100 | 10 | Medium DST Agar Klebs. 57 USA (ug/ml) 1000 | 100 | 10 | Prot. 1017 (ug/ml) 1000 | 100 | 10 | Staph. 133 (ug/ml) 1000 | 100 | 10 |
|---|---|---|---|---|---|---|---|---|---|---|---|---|---|---|---|---|
| 11 | X:N-CO-$NH_2$ R:Hyo | | 18+ | 13+ | | 13 | | | 18+ | | — | — | — | — | — | — |
| 13 | X:N-CO-$NH_2$ R:HyoHyoHyo | 19 | | | 20 | | | — | — | — | 20+ | | | — | — | — |
| 9 | X:N-CO-$NH_2$ R:HyoHyoHyoAla | | 34+ | 28+ | | 34+ | 30 | | | | — | — | — | — | — | — |
| 20 | X:N-CO-$NH_2$ R:HyoHyoHyoThr | 15+ | 14+ | | 14+ | 13+ | | — | — | — | 17+ | | | 25+ | | |
| 24 | R:O R:HyoHyoHyo | | 18 | | 18 | | | — | — | — | 18 | | | — | — | — |

+ Hemmhof mit Hintergrundwachstum

Die erfindungsgemäßen Verbindungen können somit bei der Bekämpfung bakterieller Erkrankungen dienen, besonders in Form pharmazeutischer Zusammensetzungen. Solche Pharmazeutischen Zubereitungen bestehen aus mindestens einem der erfindungsgemäßen Wirkstoffe und gegebenenfalls geeigneten nichttoxischen Träger- und Hilfsstoffen. Solche pharmazeutischen Zubereitungen gehören ebenfalls zur Erfindung.

Zur vorliegenden Erfindung gehören weiterhin pharmazeutische Zubereitungen in Dosierungseinheiten. Dies bedeutet, daß die Zubereitungen in Form einzelner Teile, z. B. Tabletten, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z. B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nichttoxischen, pharmazeutisch geeigneten Trägerstoffen sind feste, halbfeste oder flüssige Verdünnungsmittel, Füllstoffe und Formulierungshilfmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z. B. Stärken, Milchzucker, Rohrzucker, Glukose, Mannit und Kieselsäure, (b) Bindemittel, z. B. Carboxymethylcellulose, Alginate, Gelatine, Polyvinylpyrrolidon, (c) Feuchthaltemittel, z. B. Glycerin, (d) Sprengmittel, z. B. Agar-Agar, Calciumcarbonat und Natriumcarbonat, (e) Lösungsverzögerer, z. B. Paraffin und (f) Resorptionsbeschleuniger, z. B. quaternäre Ammoniumverbindungen, (g) Netzmittel, z. B. Cetylalkohol, Glycerinmonostearat, (h) Adsorptionsmittel, z. B. Kaolin und Bentonit und (i) Gleitmittel, z. B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen gegebenenfalls Opakisierungsmittel enthaltenden Überzügen und Hüllen versehen sein und auch so zusammengesetzt sein, daß sie den Wirkstoff nur oder bevorzugt in einem bestimmten Teil des Intestinaltraktes, gegebenenfalls verzögert abgeben, wobei als Einbettungsmassen z. B. Polymersubstanzen und Wachse verwendet werden können.

Der Wirkstoff kann gegebenenfalls mit einem oder mehreren der oben angegebenen Trägerstoffen auch in mikroverkapselter Form vorliegen.

Suppositorien können neben dem Wirkstoff die üblichen wasserlöslichen oder Wasserunlöslichen Trägerstoffe enthalten, z. B. Polyethylenglykole, Fette, z. B. Kakaofett und höhere Ester (z. B. $C_{14}$-Alkohol mit $C_{16}$Fettsäure) oder Gemische dieser Stoffe.

Salben, Pasten, Cremes und Gele können neben dem Wirkstoff die üblichen Trägerstoffe enthalten, z. B. tierische und pflanzliche Fette, Wachse, Paraffine, Stärke, Tragant, Cellulosederivate, Polyethylenglykol, Silicone, Bentonite, Kieselsäure, Talkum und Zinkoxid oder Gemische dieser Stoffe. Puder und Sprays können neben dem Wirkstoff die üblichen Trägerstoffe enthalten, z. B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z. B. Chlorfluorkohlenwasserstoffe enthalten.

Lösungen und Emulsionen können neben dem Wirkstoff die üblichen Trägerstoffe wie Lösungsmittel, Lösungsvermittler und Emulgatoren, z. B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe

enthalten.

Zur parenteralen Applikation können die Lösungen und Emulsionen auch in steriler und blutisotonischer Form vorliegen.

Suspensionen können neben dem Wirkstoff die üblichen Trägerstoffe wie flüssige Verdünnungsmittel, z. B. Wasser, Ethylalkohol, Proyplenglykol, Suspendiermittel, z. B. ethoxylierte Isostearylalkohole, Polyoxyethylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsmittel sowie geruchs- und geschmacksverbessernde Zusätze, z. B. Pfefferminzöl und Eukalyptusöl und Süßmittel, z. B. Saccarin enthalten.

Die erfindungsgemäßen Verbindungen sollen in den oben aufgeführten pharmezeutischen Zubereitungen vorzugsweise in einer Konzentration von etwa 0,1 bis 99,5, vorzugsweise von etwa 0,5 bis 95 Gewichtsprozent der Gesamtmischung vorhanden sein.

Die oben aufgeführten pharmazeutischen Zubereitungen können außer dem erfindungsgemäßen Wirkstoff auch weitere pharmazeutische Wirkstoffe enthalten.

Die Herstellung der oben aufgeführten pharmazeutischen Zubereitungen erfolgt in üblicher Weise nach bekannten Methoden, z. B. durch Mischen des Wirkstoffs mit dem oder den Trägerstoffen.

Zur vorliegenden Erfindung gehört auch die Verwendung der erfindungsgemäßen Wirkstoffe sowie von pharmazeutischen Zubereitungen, die den erfindungsgemäßen Wirkstoff enthalten, in der Human- und Veterinärmedizin bei der Verhütung, Besserung und/oder Heilung von Erkrankungen.

Der Wirkstoff oder die pharmazeutischen Zubereitungen können lokal, oral, parenteral, intraperitoneal und/oder rectal, vorzugsweise oral und parenteral, insbebesondere intramuskulär oder intravenös, gegebenenfalls auch als Dauertropfinfusion, appliziert werden.

Im allgemeinen empfiehlt es sich, die erfindungsgemäßen Wirkstoffe bei oraler oder parenteraler Applikation in Gesamtmengen von etwa 10 bis 1000, vorzugsweise 50 bis 600 mg/kg Körpergewicht je 24 Stunden, gegebenenfalls in Form mehrerer Einzelgaben zur Erzielung der gewünschten Ergebnisse zu verabreichen. Eine Einzelgabe enthält die erfindungsgemäßen Wirkstoffe vorzugsweise in Mengen von etwa 50 bis etwa 300 mg/kg, insbesondere 100 bis 200 mg/kg Körpergewicht.

Es kann jedoch erforderlich sein, von den genannten Dosierungen abzuweichen und zwar in Abhängigkeit von der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

**Beispiel 1** Fermentationsansatz

Der aus der DE-OS-31 02 137 bekannte Mikroorganismenstamm Streptomyces spec. WS 116 (DSM 1692) wurde in Vorkulturen in einer Nährlösung kultiviert, die sich zusammensetzte aus 2 Gew.-% Glucose, 1,3 % Hefeextrakt, 0,05 % Polyol und Leitungswasser. Der pH wurde vor dem Sterilisieren auf 6,5 eingestellt. 4 x 1000 ml-Erlenmeyerkolben, die je 150 ml dieser Nährlösung enthalten, wurden mit dem Stamm beimpft und 4 Tage bei 28°C auf einer Rundschüttelmaschine bei 220 Umdrehungen/min inkubiert. Mit diesen Vorkulturen wurde eine zweite Vorkultur in einem Labor-Fermenter, der 20 l Nährlösung enthielt, beimpft und bei 200 Umdrehungen/min, 10 l Luft/min und 28°C 3 Tage inkubiert. Mit dieser Kultur wurde ein Produktionsfermenter beimpft, der 600 l Nährlösung mit folgender Zusammensetzung enthielt:

0,7 Gew.-% Citronensäure,
0,4 Gew.-% L-Arginin, biochem.,
0,15 Gew.-% $Na_2SO_4$,
0,1 Gew.-% $MgSO_4 \cdot 7H_2O$,
0,1 Gew.-% $K_2HPO_4$,
0,05 Gew.-% $CaCO_3$,
0,01 Gew.-% $MnCl_2 \cdot 4\ H_2O$,
0,01 Gew.-% $CoCl_2 \cdot 6\ H_2O$,
0,01 Gew.-% $ZnCl_2$,
0,01 Gew.-% $FeCl_3 \cdot 6\ H_2O$,
0,05 Gew.-% Polyol (Antischaummittel) Leitungswasser

Der pH-Wert dieser Nährlösung wurde mit NaOH auf 6,2 vor der Sterilisation eingestellt.

Die Inkubation der Produktionskultur erfolgte bei 26°C bei einer Rührung von 100 Umdrehungen/min und einer Belüftung von 80 l Luft/min.

Nach etwa 3 Tagen war ein pH-Wert von 8,5 bis 8,8 erreicht. Ab diesem Zeitpunkt wurde unter sonst unveränderten Bedingungen ein konstanter pH-Wert von 7,5 durch das Zufüttern eines Feeding-Konzentrats aufrechterhalten. Das Feeding-Konzentrat bestand aus 40 Gew.-% Citronensäure, 4 Gew.-% L-Arginin und den

obengenannten Mineralsalzen in den obengenannten Konzentrationen in Leitungswasser. (Alle Nährlösungen wurden 30 Min. bei 121°C sterilisiert).

Während der Fed-Batch-Fermentation wurde zunächst die Verbindung (2) ($X=N-CO-NH_2$) gebildet. In einer späteren Phase - etwa - nach 10 Tagen, wurde das neue Tripeptid (7) gebildet. Der Endpunkt der Fermentation wurde mit Hilfe analytischer Tests ermittelt.

**Beispiel 2**

Isolierung von [$N^5$-Acetyl-$N^5$-hydroxy-L-ornithyl]-[$N^5$-acetyl-$N^5$-hydroxy-L-ornithyl]-$N^5$-acetyl-$N^5$-hydroxy-L-ornithin (7).

4000 l Verbindung (2) und Tripeptid (7) enthaltende Kulturbrühe (pH 9,06) wurden mit 50 l 1 : 1 verdünnter Salzsäure auf pH 6.2 eingestellt. Man setzte 400 g $FeCl_3 \cdot 6\ H_2O$ hinzu, rührte und gab dann 25 l verdünnte NaOH bis pH 7 unter Rühren hinzu. Dann wurde mit 200 bis 250 l/h im Westfalia-Separatur separiert. Der Überstand wurde durch eine 30 x 70 cm hoch mit Lewatit OC 1031 (ein unspezifisches Adsorptionsharz der BAYER AG) gefüllte Säule gegeben. Die Säule wurde nacheinander mit 1000 l entionisiertem Wasser und 1000 l 15 % Methanol gewaschen. Die eisen-komplexierenden Substanzen (Siderochrome) wurden mit 50 % Methanol von der Säule eluiert und in 100 l Fraktionen gesammelt. Tripeptidenthaltende Fraktionen wurden vereinigt, im Dünnschichtverdampfer auf ca. 20 l eingeengt und anschließend gefriergetrocknet. 342 g Rohprodukt wurden erhalten, anschließend in 6 l Wasser gelöst und unter Rühren mit 25 ml 50 % $FeCl_3$-Lösung versetzt. Der sich bildende Niederschlag wurde nach 15 Minuten Rühren abzentrifugiert (Hettich Rota Magna Zentrifuge, 1,5 l Becher, 30 min., 4000 Upm). Der Überstand wurde mit Schwerkraft über eine 8 x 45 cm hoch mit SP-Sephadex C25 $Fe^{+++}$ gefüllte Säule gegeben. Die Fließrate betrug 4 l pro Stunde. Man wusch die schwarz gefärbte Säule mit 5 l destilliertem $H_2O$ nach und schloß dann 10 l 0,2 m $NaH_2PO_4$/0,3 n NaCl Puffer an. Die jetzt nur noch hellbraun gefärbte Säule wurde nun mit 0,2 m $NaH_2PO_4$/0,3 m NaCl/0,05 m EDTA eluiert (Fließrate 2-3 l/h), das Säuleneluat fraktioniert in 500 ml Portionen aufgefangen. Die eisenkomplexierende Substanzen enthaltenden Fraktionen 6-14 wurden vereinigt und über eine 5 x 40 cm hoch mit Lewatit OC 1031 gefüllte Säule gegeben.

Die Fließrate betrug 2 l/h. Man wusch anschließend mit destilliertem Wasser bis mit $AgNO_3$ keine Chloridionen im Säuleneluat nachzuweisen waren (ca. 6 Liter, Fließrate 5 l/h). Anschließend eluierte man die Säule mit 3 l 90 % Methanol, die im batch aufgefangen, eingeengt und lyophilisiert wurden.

Ausbeute: 6,74 g.

Das so erhaltene Produkt wurde in 100 ml $H_2O$ gelöst auf eine 2,5 x 30 cm hoch mit Sephadex® SP 25 $H^+$ gefüllte Säule aufgetragen. Man wusch mit 1 l destilliertem $H_2O$ nach und entwickelte anschließend mit 4 l eines linearen Gradienten (0 bis 0,1 m NaCl). Das Säuleneluat wurde durch Zudosieren von 0,5 m Sörensenpuffer pH 6,5 sofort neutralisiert und in Portionen a 20 ml fraktioniert. Aliquots der Fraktionen wurden mit 0,1 m $FeCl_3$ auf Eisenkomplexbildung (Rotfärbung) geprüft. Man erhielt zwei eisenkomplexierende Fraktionen, die vereinigt wurden. Jede dieser Fraktionen wurde wie beschrieben, über je eine Lewatit OC 1031-Säule (2,5 x 30 cm) entsalzt. Man erhielt Fraktion I = 620 mg, Fraktion II = 2,3 g.

Fraktion I wurde in 5 ml $H_2O$ gelöst und auf eine 0,9 x 100 cm mit Carboxymethylcellulose (Fa. Wathmann, Typ CM 52) in der $H^+$-Form gefüllte Säule gegeben. Man entwickelte mit destilliertem Wasser. Es wurden drei mit Eisen komplexierende Verbindungen im Eluat erhalten, die für sich lyophilisiert wurden. Die zuletzt eluierte Verbindung enthielt das Tripeptid (7) und ergab 120 mg Lyophilisat.

$^1$H-NMR ($D_2O$, 250 MHz): $\delta$ = 1,54-1,96 (m; 12 H, $\beta$, $\gamma$-$CH_2$-); 2,12 (s; 9H, N-CO-$CH_3$); 3,64 (mc; 6H, $\delta$-$CH_2$-), 4,04 (t, J=7 Hz; 1N, NH-CH-); 4,14 (mc; 1H, NH-CH-); 4,40 (mc; 1H, NH-CH-).

$^{13}$C-NMR ($D_2O$, 62, 83 MHz): ppm = 18,8(3)q; 20,9 t; 21,8 t; 22,1 t; 27,5(2)t; 28,3 t; 46,6 t; 46,7 t; 47,1 t; 52,1 d; 53,1 d; 54,5 d; 168,9 s; 171,7 s; 173,5(3)s; 177,6 s.

$[\alpha]^{20}_D$: -8.57° (c = 0,567, 1 n HCl).

FAB-MS: m/z 535 ( ; M+H); $C_{21}H_{39}N_6O_{10}$.

$C_{21}H_{38}N_6O_{10}$ x $H_2O$ (552,59)

| | | | |
|---|---|---|---|
| ber.: | C 45,7 | H 7,3 | N 15,2 |
| gef.: | C 45,7 | H 7,3 | H 15,0 |

**Beispiel 3**

Die Gewinnung des Tripeptids (7) durch enzymatische Spaltung der Verbindung (2) ($X = N-CO-NH_2$)

Man löste 10 g Verbindung (2) ($X = N-CO-NH_2$) in 100 ml (100 mg/ml) 0,1 m Ammonium-carbonat-Puffer pH 7,5 und korrigierte den pH-Wert auf 7,5.

Zur Lösung setzte man 100 mg Proteinase K (Prot. K aus Pilzen, 20 mAE/mg; Fa. Merck; Best. Nr.: 140 799) hinzu. Man inkubierte über Nacht bei 37°C. Anschließend wurde das Hydrolysat 3 x im Rotationsverdampfer schonend zur Trockne eingeengt, in wenig destilliertem $H_2O$ rückgelöst und in einen Dialysierschlauch gefüllt und gegen 2 x 1 l destilliertes $H_2O$ bei 4°C über 2 x 12 h dialysiert (pro 1 g Ausgangs-Substanz je 100 ml

destilliertem $H_2O$). Die Dialysate wurden vereinigt, konzentriert und lyophilisiert.

Über eine gut gepackte 0,5 cm x 100 cm Pharmaciasäule, gefüllt mit Biogel P-2 (200-400 mesh; Bio-Rad) wurde mit einer Fließrate von 200 ml/h eine Lösung von 3 g obigem Lyophilisat in 25 ml destilliertem $H_2O$ aufgetragen. Eluiert wurde die Säule mit destilliertem $H_2O$ mit einer Fließrate von 200 ml/h. Es wurden 200 Gläser mit 8 min. Fraktionen = ca. 25 ml aufgefangen.

10-20 µl der Fraktion wurden auf Opti UP-C12 (Fluka)-Platten aufgetragen und in einem Citrat/Acetonitril Fließmittelentwickelt. (Fließmittel: Citratpuffer Merck Titrisol pH 4 auf 150 ml destilliertem Wasser verdünnen, 20 ml 0,1 m $FeCl_3$ zugeben und auf 200 ml mit Acetonitril auffüllen.

Laufstrecke: 10 cm

Anfärbung: Eisen-III-chlorid)

Als Vergleich wurden 50-100 µg Tripeptid (7) mit aufgetragen.

Die tripeptidhaltigen Fraktionen wurden vereinigt, konzentriert und lyophilisiert. Ausbeute 500-700 mg Tripeptid (7).

Das so gewonnene Tripeptid (7) besaß neben identischen $^1H$-; $^3C$-NMR-spektroskopischen Daten (siehe Beispiel 2) die spezifische Drehung $[\alpha]^D{}_{20}$- 8,63° (c = 1,008, 1n HCl).

**Beispiel 4**

Nucleosid (4) X = N-CO-$NH_2$, aus Verbindung (2) X = N-CO-$NH_2$

In zwei Parallelansätzen wurden jeweils 1,5 g (1,5 mmol) Verbindung (2) (X = N-CO-$NH_2$) in 500 ml bidestilliertem $H_2O$ gelöst und mit 0,5 m Trispuffer auf pH 7,2 eingestellt. Den Lösungen wurden jeweils 0,75 ml (46,9 U) Leucinaminopeptidase (mikrosomal, Hersteller: Sigma L 5006; spez. Aktivität 10-20 U/mg, 37°C, Leucinamid als Substrat) zugegeben und bei 37°C gerührt. Leucinaminopeptidase wurde zu den Ansätzen nach 16 h (28,1 U), 5 Tagen (12,5 U) und 6 Tagen (12,5 U) zugegeben. Nach 7 Tagen war die Reaktion beendet. Die Ansätze wurden vereinigt und gefriergetrocknet. Das Lyophilisat wurde an 300 ml Sephadex SP 25 im Kältelabor (4°C) chromatographiert (Vorlauf 700 ml bidestilliertes Wasser, anschließend Gradient 0,01 - 0,5 n NaCl-Lösung, Fluß 5 ml/min., Fraktionsgröße 20 ml). In den Auslauf wurde 0,5 m Sörensenpuffer gepumpt, so daß die gesammelten Fraktionen pH 6,7 aufwiesen. Nach HPLC und DC-Analyse wurden die identischen Fraktionen zusammengefaßt und an Lewatit LGP 4067 entsalzt.

| | | |
|---|---|---|
| Fr. 131-140 | 41,8 mg | |
| Fr. 164-224 | 626,8 mg | 53,7% |
| Fr. 228-235 | 126,4 mg | |

$^1H$-NMR ($D_2O$, 250 MHz): δ = 3,39 (s; 3H, N-$CH_3$); 3,98 (d, J = 3,8 Hz; 1H, H6'); 4,07 (dd, J = 6,5 Hz; J = 4,8 Kz; 1H, H-4'); 4,36-4,48 (m, 2H,H-2',3'); 4,55 (dd, J = 6,5 Kz, J = 3,8 Hz, 1H, H-5'); 5,92 (d, J = 4,0 Kz; 1H, H-1'); 6,48 (d, J = 8,0 Hz; 1H, H-5); 8,36 (d, J = 8,0 Hz; 1H, H-6).

$^{13}C$-NMR ($D_2O$, Dioxan als Standard, 50,32 MHz): ppm = 170,4 (s; C-7'); 166,8 (s; N-CO-$NH_2$); 155,1 (s, Pyrimidin C-4); 151,9 (s, Pyrimidin C-2); 137,0 (d, Pyrimidin C-6); 96,0 (d, Pyrimidin C-5); 80,0 (d); 74,5 (d) ; 67,3 (d); 65,3 (d); 57,3 (d, C-6'); 51 ,4 (d, C-4'); 29,3 (qu, N-$CH_3$).

UV (1 n HCl) : $\lambda_{max}$ = 214 (10490), 235 (8205), 305 (15306).

$[\alpha]^{20}_D$ - 33,61 ± 0,08 (c = 1,02; $H_2O$).

FAB-MS: m/z = 390 (6,7 %, M + H).

$C_{13}H_{19}N_5O_7S$ x $H_2O$ (MG 407,40)

| | | | | | |
|---|---|---|---|---|---|
| ber.: | C 38,2 | H 5,2 | N 17,2 | O 31,4 | S 7,9 |
| gef.: | C 38,2 | H 5,1 | N 16,8 | O 31,7 | |

**Beispiel 5**

Nucleosid (4) aus Verbindung (3) X = N-CO-$NH_2$

Man löste 10 g Verbindung (2) in 100 ml 0,1 m Ammoniumcarbonat-Puffer pH 7,5. (Konzentration 100 mg/ml)

Der pH-Wert wurde auf 7,5 korrigiert. Zur Lösung setzte man 1500 U/mg Schweine-Nieren-LAP zu. Man inkubierte 3 Tage bei 37°C im Brutschrank. Das Inkubat wurde dann im Rotationsverdampfer 3 x zur Trockne eingeengt, in wenig destilliertem $H_2O$ rückgelöst und in einen Dialysierschlauch gefüllt. Man dialysierte 2 x über Nacht im Kältelabor gegen destilliertes $H_2O$ mit dem 20-fachen Ausgangsvolumen. Das Dialysat wurde im Rotationsverdampfer konzentriert und lyophilisiert.

Ausbeute: Man erhielt 9-10 g Rohmaterial mit 70 bis 90 % Nucleosid (4) X = N-CO-$NH_2$.

Zur Reinigung trug man je 3 g des rohen serinfreien Nucleosids (4) auf eine 5 x 90 cm mit Biogel P 2 in destilliertem $H_2O$ gefüllte Säule auf. Man entwickelte mit destilliertem $H_2O$ und vereinigte die nucleosidhaltigen Fraktionen. Nach konzentrieren wurden sie lyophilisiert. Ausbeute 1,8-2 g des serinfreien Nucleosids (4).

Alle spektroskopischen Daten stimmten mit denen aus Beispiel 4 überein.

**Beispiel 6**

Nucleosid (4) X = O, R = H

1,5 g (1,6 mmol) Verbindung 2 (X = O) wurden in 500 ml bidestilliertem Wasser gelöst und mit 0,5 m Trispuffer auf pH 7,2 eingestellt. Der Lösung wurden 0,75 ml (46,9 U) Leucinaminopeptidase (mikrosomal, Hersteller: Sigma L. 5006; spezifische Aktivität 10-20 U/mg; 37°C, Leucinamid als Substrat) zugegeben und bei 37°C gerührt. Leucinaminopeptidase wurde zu dem Ansatz nach 16 h (25 U), 2 Tagen (25 U) und 3 Tagen (25 U) zugegeben. Nach 7 Tagen war die Reaktion beendet. Die Lösung wurde gefriergetrocknet und an 300 ml Lewatit LGP 4067 (6) entsalzt. Mit bidestilliertem Wasser wurde solange gewaschen, bis UV-aktives Material eluiert wurde. Das restliche Produkt wurde mit Methanol: Wasser 1 : 1 eluiert.

| | | |
|---|---|---|
| Fr. 11-29 | 1,7 g | Salz und Hydroxamsäure (5) |
| Fr. 30-47 | 544 mg | (4) (99,4 %) |

Fp.: 105-10°C (dec.)

$^1$H-NMR ($D_2O$ 250 MHz): δ = 3,29 (s; 3H, N-$CH_3$) ; 3,94 (d, J = 4 Hz; 1H, H-6'); 4,04 (dd, J = 6 Hz; 1H, H-4'); 4,36-4,50 (m; 2H, H-2',3'); 4,56 (dd, J = 6 Hz, J = 4 Hz; 1H, H-5'); 5,94 (d, J = 6 Hz; 1H, H-1'); 5,96 (d, J = 8 Hz; 1H, H-5); 8,45 (d, J = 8 Hz; 1H, H-6).

$^{13}$C-NMR ($D_2O$, Dioxan als Standard, 50,32 MHz): ppm = 170,0 (s; C-7'); 164,9 (s, Pyrimidin C-4); 152,2 (s, Pyrimidin C-2); 141,5 (d, Pyrimidin C-6); 100,1 (d, Pyrimidin C-5); 80,4 (d); 74,7 (d); 67,6 (d); ca. 65 (d) (Überlagerung von Dioxan); 56,9 (d, C-6'); 52,0 (d, C-4'); 27,4 (qu, N-$CH_3$).

UV (1 n HCl): $\lambda_{max}$ = 210 (8383); 266 (7097).

$[\alpha]^{20}_D$: + 3,69 ± 0,08° (c = 0,994; $H_2O$).

FAB-MS: m/z = 348 (3,3 %; M + H).

$C_{12}H_{17}N_3O_7S$ x $H_2O$ (MG 365,36)

| | | | | | |
|---|---|---|---|---|---|
| ber.: | C 39,4 | H 5,2 | N 11,5 | O 35,0 | S 8,8 |
| gef.: | C 38,6 | H 5,2 | N 11,5 | O 36,0 | |

**Beispiel 7**

Hydroxamsäure (5)

4,9 g eines Gemisches aus Salz und Hydroxamsäure (5) aus der Darstellung von (4) X = O wurden an 200 ml QAE Sephadex® A 25 (OH-Form) chromatographiert. Nach Waschen mit 1,1 l bidestilliertem Wasser wurde mit 0,1 n HCl eluiert. Die mit Eisen-III-chlorid anfärbenden Fraktionen (DG einheitlich) wurden vereinigt und aus Ethanol/Wasser kristallisiert. Es wurden 1,25 g kristalline Hydroxamsäure (5) erhalten.

Fp.: 221°C (dec).

$[\alpha]^{20}_D = + 3,37$ (c = 1,005; $H_2O$).

$^1$H-NMR ($D_2O$, 250 MHz): δ = 1,63-1,92 (m; 4H, $CH_2$-$CH_2$-); 2,12 (s; 3H, N-$COCH_3$); 3,65 (mc; 2H, N-$CH_2$-); 3,74 (mc; 1H, N-CH).

MS (70 eV): m/z = 190 (3 %, M+), 145 (5 %), 86 (19 %), 43 (100 %).

| | | | | |
|---|---|---|---|---|
| $C_7H_{14}N_2O_4$ (190,2) | ber.: | C 44,2 | H 7,4 | N 14,7 |
| | gef.: | C 44,1 | H 7,4 | H 14,7 |

**Beispiel 8**

Alanylnucleosid (8)

100 mg (0,26 mmol) serinfreies Nucleosid (4) X = N-CO-$NH_2$ und 21,8 mg (0,26 mmol) Natriumhydrogencarbonat wurden in 10 ml Wasser/Tetrahydrofuran 1 : 1 gelöst. Nach Zugabe von 147,1 mg (0,51 mmol) BOC-L-Alaninhydroxysuccinimidester wurde bei 25°C 16 h gerührt. Anschließend wurden nochmals 21,8 mg (0,26 mmol) Natriumhydrogencarbonat und 73,6 mg, (0,26 mmol) der aktivierten Aminosäure zugegeben. Nach weiteren 16 h wurde die Lösung eingeengt und im Hochvakuum getrocknet. Der Rückstand wurde in wenig Wasser aufgenommen und mit 2 n Natronlauge auf pH 7 eingestellt. Chromatographiert wurde an 40 ml Sephadex® 25 H⊕ (Säulendurchmesser: 1,5 cm; Laufmittel: stufenloser Gradient 500 ml $H_2O$ → 0,1 n NaCl). Die Substanz enthaltenden Fraktionen wurden gefriergetrocknet und anschließend an 30 ml Lewatit® LGP 4067 entsalzt. Es wurden 54,7 mg (46,3 %) des Alanylnucleosids (8) nach Gefriertrocknung erhalten Physikalische Daten siehe Tabelle 3.

**Beispiel 9**

Peptidylnucleosid (9)

57 mg (0,55 mmol) [$N^5$-Acetyl-$N^5$-O-benzyl-$N^2$-carboxy-benzyl-L-ornithyl]-[$N^5$-acetyl-$N^5$-O-benzyl-L-ornithyl]-$N^5$-acetyl-$N^5$-O-benzyl-L-ornithin (Derivat der Trihydroxamsäure (2)) wurden in 5 ml wasserfreiem Tetrahydrofuran gelöst und auf -20°C gekühlt. Nach Zugabe von 0,061 ml (0,55 mmol) N-Methylmorpholin wurden 0,068 ml (0,55 mmol) Chlorameisensäureisobutylester in 1 ml Wasserfreiem Tetrahydrofuran zugegeben. Nach 30 min. Aktivierung bei -20°C wurden 100 mg (0,22 mmol) Alanylnucleosid (8) in 5 ml THF/$H_2O$ 1 : 1 schnell zugetropft und 16 h unter Auftauen auf 25°C gerührt. Die Lösung wurde eingeengt, in Wasser aufgenommen und mit 1 n HCl auf pH 2 eingestellt. Nach mehrfacher Extraktion mit Essigsäureethylester/Tetrahydrofuran 1 : 1 (insgesamt 60 ml) wurden nach Trocknen und Einrotieren 510 mg Rohsubstanz erhalten (N, O blockiert). Dieses Rohmaterial wird in 50 ml Ethanol/Wasser 6 : 4 (Volumenteile) gelöst und über 100 ml Palladium/Kohle mit Hilfe eines Vibromischers hydriert (25°C, Normaldruck). Nach 3 h wurden 100 mg Palladium/Kohle, nach 6 h 50 mg Palladium/Kohle zugesetzt. Nach 9 h war die Deblockierung beendet. Der Katalysator wurde heiß abfiltriert und mehrfach mit Ethanol/Wasser 1 : 1 nachgewaschen. Das Filtrat wurde teilweise eingeengt und anschließend gefriergetrocknet. 310 mg des deblockierten Rohmaterials wurden an 30 ml Sephadex-SP 25 H⊕ (Säulendurchmesser: 1,5 cm; Laufmittel: 100 ml Wasser; stufenloser Gradient 800 ml $H_2O$ → 0,1 n NaCl; Fluß 2,5 ml/min.; Fraktionsgröße 10 ml) chromatographiert. Nach Entsalzen der einheitlichen Fraktionen an Lewatit® LGP 4067 wurden 11,5 mg des Peptidylnucleosids (9) erhalten. Physikalische Daten siehe Tabelle 3.

Die Verbindungen 10 bis 33 wurden in Analogie zu den Beispielen 5 und 6 hergestellt. Physikalische Daten siehe Tabelle 3.

**Tabelle 3**

| X: $N\text{-}CO\text{-}NH_2$ R: | $^{1}H$-NMR ($D_2O$), 250 MHz, HDO als interner Standard 4.80 ppm) δ [ppm]; J = Linien-abstände (Hz) | UV (qualitativ, 0,1 n HCl) $\lambda_{max}$ (nm) | Summenformel | FAB-MS (Fast Atom Bombard-ment) |
|---|---|---|---|---|
| 8 Ala | 1.56 (d, J=7.5 Hz; 3H, $-CH-CH_3$); 4.13 (qu, J=7.5 Hz, 1N, N-CH-). | 304 | $C_{16}H_{24}N_6O_8S$ | |
| 9 HyoHyoHyoHyoAla | 1.39 (d, J=7.1 Hz; 3H, $-CH-CH_3$); 1.56-1.98 (m; 12H, $-CH_2-$); 2.13 (s; 9H, $N-CO-CH_3$); 3.67 (mc; 6H,$N-CH_2-$). | 305 | $C_{37}H_{60}N_{12}O_{17}S$ | M-H 975 (1 %) |
| 10 His | 3.17-3.43 (m; 3H, $N-CH-CH_2-C=$); 7.40 (s; 1H, N-CH-C); 8.59 (s; 1H, N-CH=N-). | 305 | $C_{19}H_{26}N_8O_8S$ | |
| 11 Hyo | 1.62-2.04 (m; 4H, $-CH_2-$); 2.14 (s; 3H, 304 $N-CO-CH_3$); 3,67 (mc; 2H, $N-CH_2-$); 4.09 (t, J=6 Hz; 1H, N-CH-). | 304 | $C_{20}H_{21}N_7O_{10}S$ | |
| 12 HyoHyo | 1.69-2.07 (m; 8H, $-CH_2-$); 2.22 (s; 6H, $N-CO-CH_3$); 3.73 (mc; 4H, $N-CH_2-$); 4.14 (t, J=6 Hz; 1H, N-CH-). | 306 | $C_{27}H_{43}N_9O_{13}S$ | |
| 13 HyoHyoHyo | 1.61-1.97 (m; 12H, $-CH_2-$); 2,15 (s, 9H, $N-CO-CH_3$); 3.67 (mc; 6H, $N-CH_2-$); 4.08 (t, J=6 Hz; 1H, N-CH-). | 306 | $C_{34}H_{55}N_{11}O_{16}S$ | M-H 904 (1 %) |
| 14 AlaSer | 1.56 (d, J=7.5 Hz; $-CH-CH_3$); 4.14 (qu, J=7.5 Hz; 1H, N-CH-). | 305 | $C_{19}H_{29}N_7O_{10}S$ | |
| 15 HisSer | 3.43 (mc; 2H, $-CH_2-C=$); 7.41 (s; 1H, N-CH=C); 8.63 (s; 1H; N-CH=N). | 305 | $C_{22}H_{31}N_9O_{10}S$ | |
| 16 HyoSer | 1.71-2.07 (m; 4H, $-CH_2$); 2.20 (s; 3H, $N-CO-CH_3$); 3.72 (mc; 2H, $N-CH_2-$); 4.17 (t, J=6.2 Hz; 1H, N-CH-). | 304 | $C_{23}H_{36}N_8O_{12}S$ | |
| 17 HyoHyoSer | 1.65-1.97 (m; 8H, $-CH_2-$); 2.15 (s, 6H, $N-CO-CH_3$); 3.68 (mc; 4H, $N-CH_2-$); 4.08 (t, J=6 Hz; 1H, N-CH-). | 306 | $C_{30}H_{48}N_{10}O_{15}S$ | |
| 18 ThrSer | 1.31 (d, J=6 Hz; 3H, $CH-CH_3$); 3.95 (d, J=6 Hz; 1H, N-CH-); 4.12 (qui, J=6 Hz; 1H, -O-CH-). | 305 | $C_{20}H_{31}N_7O_{10}S$ | |
| 19 Thr | 1.31 (d, J=6 Hz; 3H, $CH-CH_3$); 3.94 (d, J=6 Hz; 1H, N-CH-); 4.19 (qui, J=6 Hz; 1H, -O-CH). | 304 | $C_{17}H_{26}N_6O_9S$ | |
| 20 HyoHyoHyoThr | 1.21 (d, J=6 Hz; 3H, $CH-CH_3$); 1.45-1.99 (m; 12H, $-CH_2-$); 2.15 (s; 9H, $N-CO-CH_3$); 3.68 (mc; 6H, $-CH_2-$); 4.08 (t, J=5 Hz; 1H, N-CH-); 4.28 (qu, d, J=6 Hz; 1H, N-CH-). | 306 | $C_{38}H_{62}N_{12}O_{18}S$ | M-H 1005 (1.5 %) |
| 21 Ala | 1.63 (d, J=6 Hz; 3H, $-CH-CH_3$); 4.21 (qu; J=6 Hz; 1H, N-CH-). | 265 | $C_{15}H_{22}N_4O_8S$ | |
| 22 Hyo | 1.62-2.00 (m; 4H, $-CH_2-$); 2.12 (s; 3H, $N-CO-CH_3$); 3.65 (mc; 2H, $N-CH_2-$); 4.07 (t, J=6 Hz; 1H, N-CH-). | 265 | $C_{19}H_{29}N_5O_{10}S$ | |
| 23 HyoHyo | 1.62-2.00 (m; 8H, $-CH_2-$); 2.16 (s; 6H, $N-CO-CH_3$); 3.68 (mc; 4H, $N-CH_2-$); 4.09 (t, J=6 Hz; 1H, N-CH-). | 263 | $C_{26}H_{41}N_7O_{13}S$ | |

| | | | | |
|---|---|---|---|---|
| 24 HyoHyoHyo | 1.56-1.96 (m; 12H, $-CH_2-$);<br>2.14 (s; 9H, $N-CO-CH_3$);<br>3.67 (mc; 6H, $N-CH_2-$);<br>4.07 (t, J=6 Hz; 1H, N-CH-). | 264 | $C_{33}H_{53}N_9O_{16}S$ | M-H 862 (1 %) |
| 25 AlaSer | 1.65 (d, J=7.5 Hz; 3H, $CH-CH_3$);<br>4.23 (qu, J=7.5 Hz; 1H, N-CH-). | 265 | $C_{18}H_{27}N_5O_{10}S$ | |
| 26 HisSer | 3.36 (dd, J=15 Kz; J=7.5 Hz;<br>1H, $N-CH-CH_2$); 3.46 (dd, J=15 Hz;<br>J=5 Hz; 1H, $-N-CH-CH_2-$);<br>3.76-3.90 (m; 1H, N-CH);<br>7.38 (s; 1H, N-CH=C-);<br>8.62 (s; 1H, N-CH=N-). | 266 | $C_{21}J_{29}H_7O_{10}S$ | |
| 27 HyoSer | 1.67-2.03 (m; 4H, $-CH_2-$);<br>2.15 (s; 3H, $N-CO-CH_3$);<br>3.62-3.76 (m; 2H, $N-CH_2-$);<br>4.13 (t, J=6 Hz; 1H, N-CH-). | 265 | $C_{22}H_{34}N_6O_{12}S$ | |
| 28 HyoHyoSer | 1.65-2.09 (m; 8H, $-CH_2$);<br>2.19 (s; 6H, $N-CO-CH_3$);<br>3.71 (mc; 4H, $N-CH_2-$);<br>4.11 (t, J=6 Hz; 1H, N-CH-). | 265 | $C_{29}H_{46}N_8O_{15}S$ | |
| 29 ThrSer | 1.35 (d, J=7 Hz; 3H, $-CH-CH_3$);<br>4.01 (d, J=5.8 Hz; 1H, N-CH-). | 264 | $C_{19}H_{29}N_5O_{11}S$ | |
| 30 Thr | 1.37 (d, J=6 Hz; 3H, $-CH-CH_3-$);<br>3.97 (d, J=6.9 Hz; 1H, N-CH-);<br>4.24 (qui, J=6 Hz; 1H, -O-CH). | 264 | $C_{16}H_{24}N_4O_9S$ | |
| 31 HyoHyoHyoThr | 1.19 (d, J=6 Hz; 3H, $-CH-CH_3$);<br>1.55-1.97 (m; 12H, $-CH_2-$);<br>2.15 (s; 9H, $N-CO-CH_3$);<br>3.66 (mc; 6H, $N-CH_2$);<br>4.06 (t, J=6 Hz; 1H, N-CH-);<br>4.27 (qu, d. J=6 Hz;<br>J=5 Hz; 1H, $O-CH-CH_3$). | 266 | $C_{37}H_{60}N_{10}O_{18}S$ | |

**Patentansprüche** für die Vertragsstaaten BE, CH, DE, FR, GB, IT, LI, NL, SE

1. Verbindungen der allgemeinen Formel (1)

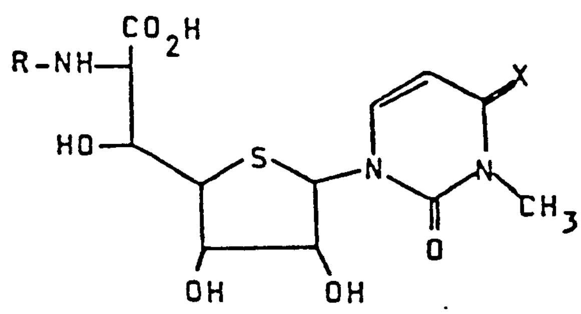

(1)

in der

X 0 oder $N-CO-NH_2$ und

R den Rest einer Aminosäure (außer Serin), einen Oligopeptidrest (außer HyoHyoHyoSer und seiner Derivate an der freien Aminogruppe) und einen Rest aus der Gruppe HyoHyoSer, HyoHyoHyoAla, HyoHyoHyoThr, Hyo, HyoHyo oder HyoHyoHyo darstellt, wobei Hyo die Bedeutung $N^5$-Acetyl-$N^5$-hydroxy-L-ornithin hat.

2. Verbindungen nach Anspruch 1, dadurch gekennzeichnet, daß

R Glycin, Alanin, Valin, Leucin, Isoleucin, Phenylalanin, Tyrosin, Prolin, Hydroxyprolin, Threonin, Cystein, Cystin, Methionin, Tryptophan, Aspargaginsä:ıre, Asparagin, Glutaminsäure, Glutamin, Hydroxyglutaminsäure, Arginin, Lysin, Histidin oder ein Oligopeptidrest aus diesen Aminosäure, darstellt.

3. Verbindungen nach Anspruch 1 oder 2 in denen R Alanin oder Threonin darstellt.

4. Verbindung der Formel (4)

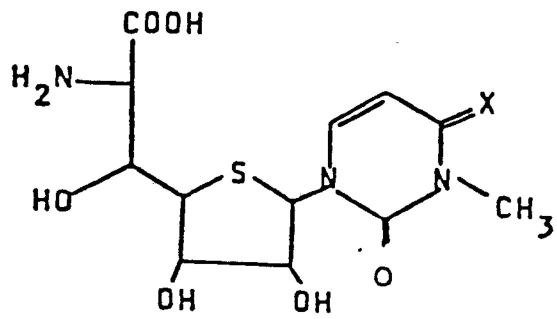

(4)

in der X Sauerstoff oder N-CO-$NH_2$ darstellt.

5. Verfahren zur Herstellung von Verbindungen gemäß Anspruch 1 dadurch gekennzeichnet, daß man Verbindungen der Formel (2)

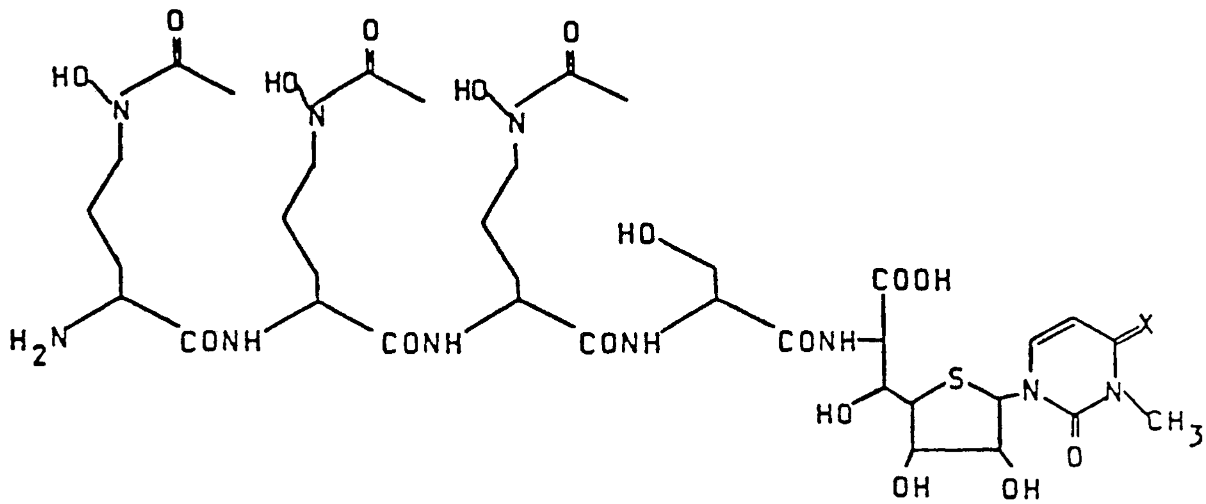

(2)

in der X = 0 oder N-CO-$NH_2$ ist,
mit geeigneten Enzymen zu Verbindungen der Formel (3)

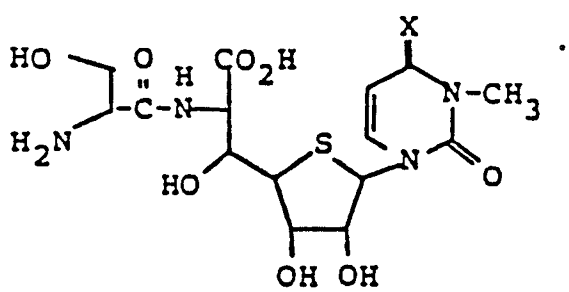

(3)

und anschließend mit mikrosomaler Leucinaminopeptidase zu Verbindungen der Formel (4)

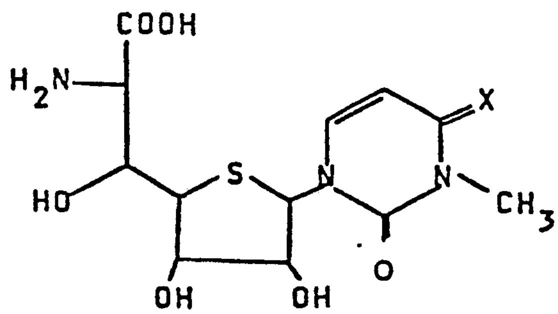

(4)

spaltet,

oder die Verbindungen der Formel (2) mit mikrosomaler Leucinaminopeptidase direkt zu den Verbindungen der Formel (4) spaltet

und anschließend die Verbindungen der Formeln (3) und (4) mit einer Aminosäure, die dem Rest R in Anspruch 1 entspricht oder deren aktiviertem Derivat verknüpft.

6. Arzneimittel enthaltend mindestens eine Verbindung der allgemeinen Formel 1 gemäß Aspruch 1.

7. Verbindungen der allgemeinen Formel (1) nach Anspruch 1 zur Verwendung in einem Verfahren zur therapeutischen Behandlung des menschlichen oder tierischen Körpers.

8. Verwendung von Verbindungen der allgemeinen Formel (1) nach Anspruch 1 zur Herstellung von Arzneimitteln.

**Patentansprüche** für den Vertragsstaat AT

1. Verfahren zur Herstellung der Verbindungen der Formel (1)

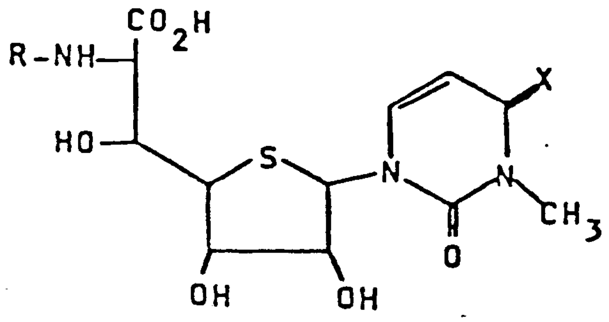

(1)

in der

X  0 oder N-CO-$NH_2$ und

R  den Rest einer Aminosäure (außer Serin), einen Oligopeptidrest (außer HyoHyoHyoSer und seiner Derivate an der freien Aminogruppe) und einen Rest aus der Gruppe HyoHyoSer, HyoHyoHyoAla, HyoHyoHyoThr, Hyo, HyoHyo oder HyoHyoHyo darstellt, wobei Hyo die Bedeutung $N^5$-Acetyl-$N^5$-hydroxy-L-ornithin hat,

dadurch gekennzeichnet, daß man eine Verbindung der Formel (2)

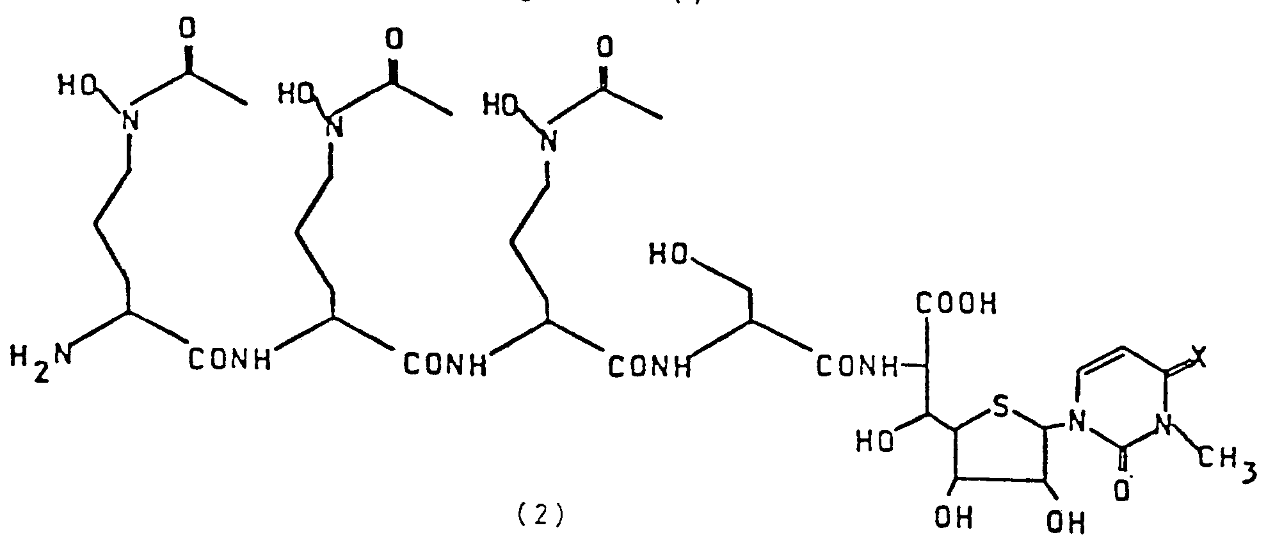

(2)

in der

X  die Bedeutung 0 oder N-CO-$NH_2$ hat, mit einem geeigneten Enzym zu den Verbindungen (3) oder (4)

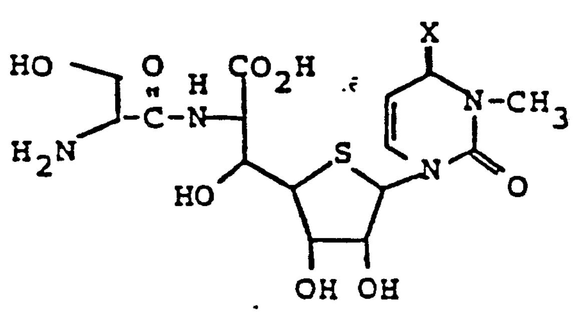

(3)

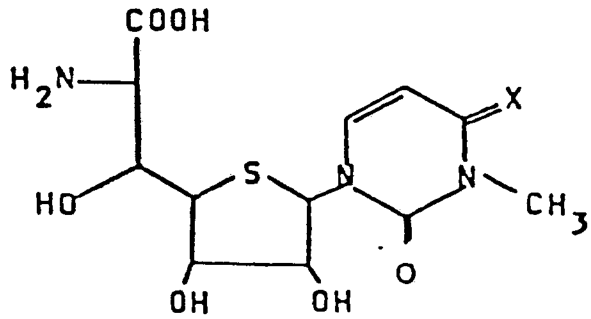

(4)

in denen
X die Bedeutung 0 oder $N-CO-NH_2$ hat,
spaltet, und die Verbindungen (3) oder (4) mit den dem Rest R entsprechenden blockierten Oligopeptiden oder Aminosäuren kuppelt und anschließend deblockiert.

2. Verfahren nach Anspruch 1, dadurch gekennzeichnet, daß man die Spaltung von (2) nach (3) Peptidhydrolasen der Gruppen EC 3.4.11 (α-Amino-acyl-peptidhydrolasen) und EC 3.4.21-23 (Proteasen) verwendet.

3. Verfahren nach Anspruch 2, dadurch gekennzeichnet, daß man die Spaltung von (2) nach (3) mit α-Chymotrypsin, Subtilisin, Aminoacylase I, β-Lactamase aus E Coli T7, Proteinase K oder Pronase K durchführt.

4. Verfahren nach Anspruch 1, dadurch gekennzeichnet, daß man die Spaltung von (2) nach (4) mit mikrosomaler Leucinaminopeptidase durchführt.

5. Verfahren nach Anspruch 4, dadurch gekennzeichnet, daß man die Spaltung in einem wäßrigen Medium bei einem pH-Wert von 6,6 - 7,8 und bei einer Reaktionstemperatur von 20 - 40° durchführt.

6. Verfahren nach Anspruch 1, dadurch gekennzeichnet, daß die eingesetzten Aminosäuren oder Peptide, die dem Rest R entsprechen, N-blockiert sind.

7. Verfahren nach Anspruch 6, dadurch gekennzeichnet, daß die Aminosäuren und Peptide, die dem Rest R entsprechen, durch t-Butyloxycarbonyl- oder Benzyloxylcarbonyl-Gruppe blockiert sind.

8. Verfahren nach Anspruch 1, dadurch gekennzeichnet, daß die Peptid- oder Oligopeptid-Verknüpfung mit den Verbindungen der Formeln (3) und (4) nach der Mixed-Anhydrid-Methode erfolgt.

9. Verfahren nach Anspruch 1, dadurch gekennzeichnet, daß die Kupplung mit den Verbindungen (3) und (4) in einem wäßrig organischen Medium, insbesondere in Wasser/THF-Gemischen vorgenommen wird.

**Claims** for the contracting states BE, CH, DE, FR, GB, IT, LI, NL, SE

1. Compounds of the general formula (1)

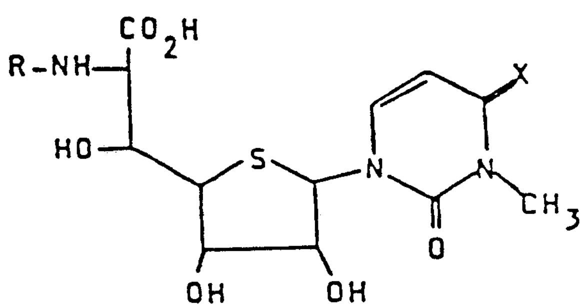

(1)

in which
X represents 0 or $N-CO-NH_2$ and
R represents the radical of an aminoacid (with the exception of serine), an oligopeptide radical (with the exception of HyoHyoHyoSer and its derivatives on the free amino group) or a radical from the group comprising HyoHyoSer, HyoHyoHyoAla, HyoHyoHyoThr, Hyo, HyoHyo or HyoHyoHyo,
wherein
Hyo denotes $N^5$-acetyl-$N^5$-hydroxy-L-ornithine.

2. Compounds according to Claim 1, characterised in that
R represents glycine, alanine, valine, leucine, isoleucine, phenylalanine, tyrosine, proline, hydroxyproline, threonine, cysteine, cystine, methionine, tryptophan, asparaginic acid, asparagine, glutamic acid, glutamine, hydroxyglutamic acid, arginine, lysine or histidine, or an oligopeptide radical of these aminoacids.

3. Compounds according to Claim 1 or 2, in which
R represents alanine or threonine.

4. Compound of the formula (4)

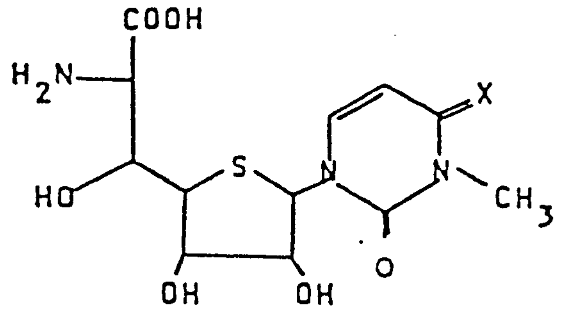

(4)

in which

X represents oxygen or N-CO-$NH_2$.

5. Process for the preparation of compounds according to Claim 1, characterised in that compounds of the formula (2)

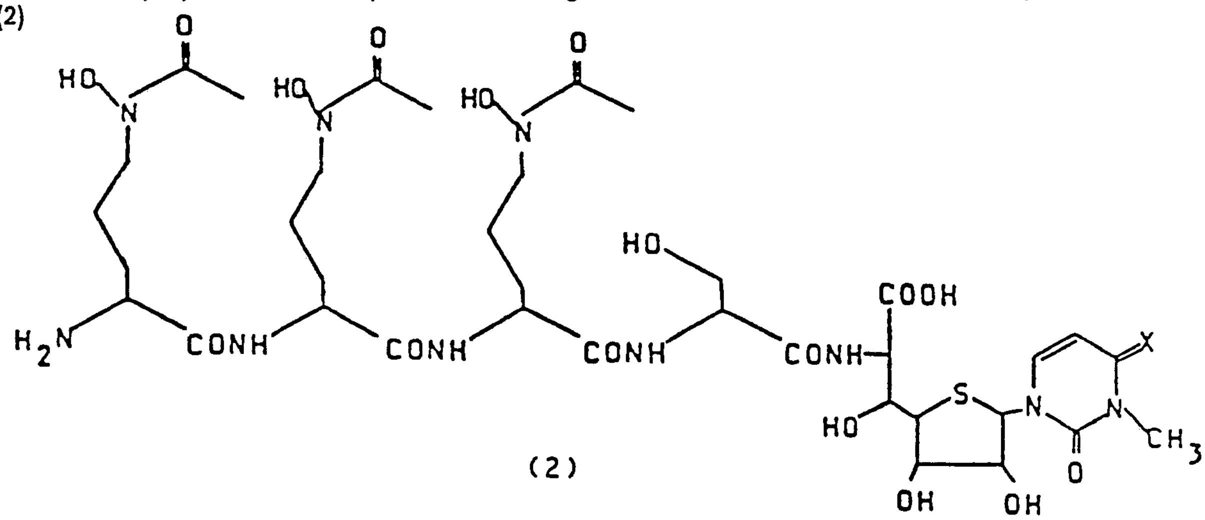

(2)

in which

X is 0 or N-CO-$NH_2$,

are split with suitable enzymes to give compounds of the formula (3)

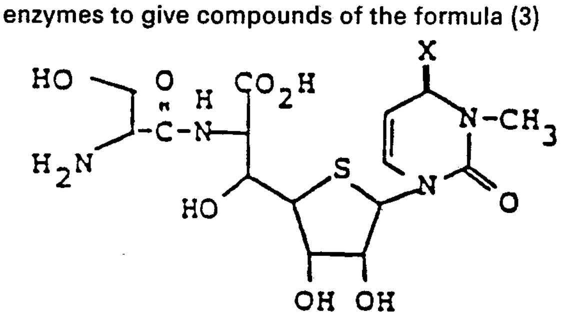

(3)

and then with microsomal leucine aminopeptidase to give compounds of the formula (4)

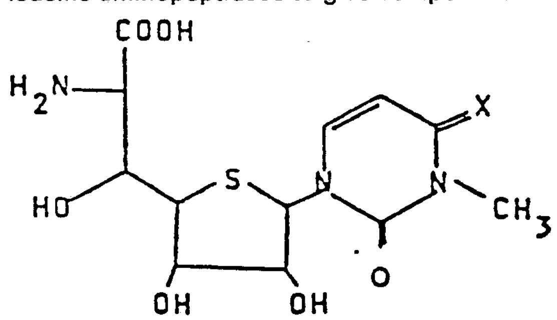

(4)

or the compounds of the formula (2) are split directly with microsomal leucine aminopeptidase to give compounds of the formula (4),

and the compounds of the formulae (3) and (4) are then linked with an aminoacid which corresponds to the radical R in Claim 1, or an activated derivative thereof.

6. Medicaments containing at least one compound of the general formula 1 according to Claim 1.

7. Compounds of the general formula (1) according to Claim 1 for use in a process for the therapeutic treatment of the human or animal body.

8. Use of compounds of the general formula (1) according to Claim 1 for the preparation of medicaments.

**Claims** for the contracting state AT

1. Process for the preparation of compounds of the formula (1)

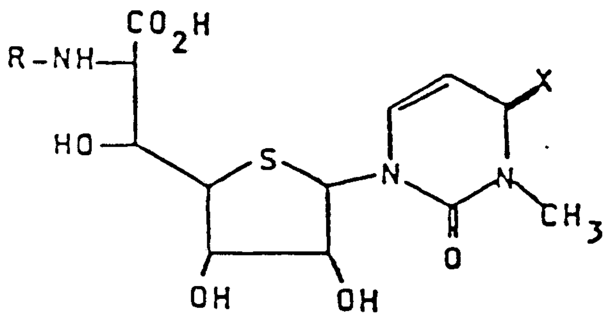

(1)

in which

X represents 0 or N-CO-$NH_2$ and

R represents the radical of an aminoacid (with the exception of serine), an oligopeptide radical (with the exception of HyoHyoHyoSer and its derivatives on the free amino group) or a radical from the group comprising HyoHyoSer, HyoHyoHyoAla, HyoHyoHyoThr, Hyo, HyoHyo or HyoHyoHyo,

wherein

Hyo denotes $N^5$-acetyl-$N^5$-hydroxy-L-ornithine, characterised in that a compound of the formula (2)

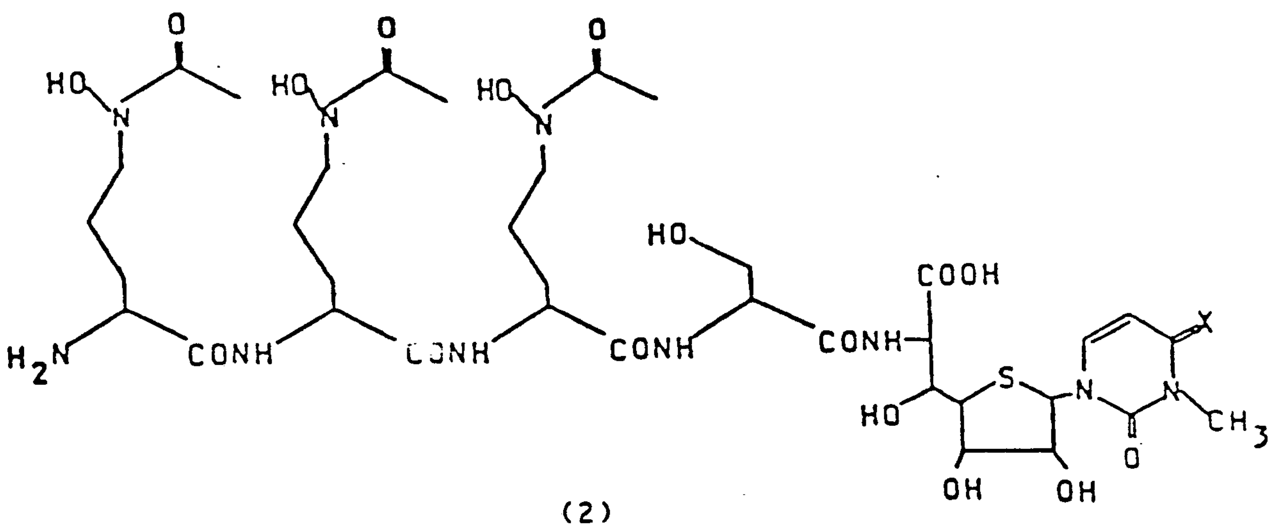

(2)

in which

X denotes 0 or N-CO-$NH_2$,

is split with a suitable enzyme to give the compounds (3) or (4)

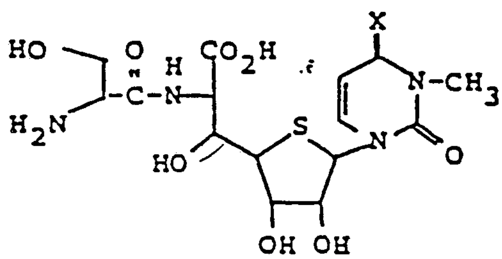

(3)

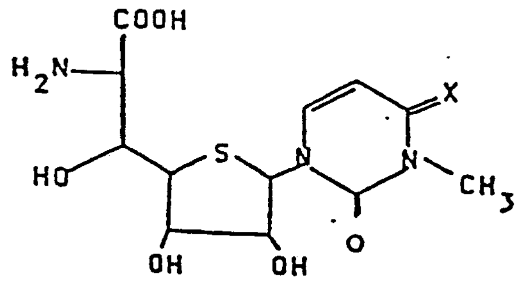

(4)

in which

X denotes 0 or N-CO-$NH_2$,

and the compounds (3) or (4) are coupled with the blocked oligopeptides or aminoacids corresponding to the radical R and are then deblocked.

2. Process according to Claim 1, characterised in that peptide hydrolases of groups EC 3.4.11 (α-amino-acyl-peptide hydrolases) and EC 3.4.21-23 (proteases) are used for the splitting of (2) to (3).

3. Process according to Claim 2, characterised in that the splitting of (2) to (3) is carried out with α-chymotrypsin, subtilisin, aminoacylase I, β-lactamase from E coli T7, proteinase K or pronase K.

4. Process according to Claim 1, characterised in that the splitting of (2) to (4) is carried out with microsomal leucine aminopeptidase.

5. Process according to Claim 4, characterised in that the splitting is carried out in an aqueous medium at a pH value of 6.6 - 7.8 and at a reaction temperature of 20 - 40°.

6. Process according to Claim 1, characterised in that the aminoacids or peptides used which correspond to the radical R are N-blocked.

7. Process according to Claim 6, characterised in that the aminoacids or peptides which correspond to the radical R are blocked by a t-butoxycarbonyl or benzyloxycarbonyl group.

8. Process according to Claim 1, characterised in that the peptide or oligopeptide linkage with the compounds of the formulae (3) and (4) is effected by the mixed anhydride method.

9. Process according to Claim 1, characterised in that the coupling with the compounds (3) and (4) is carried out in an aqueous organic medium, in particular in water/tetrahydrofuran mixtures.

**Revendications** pour l'Etat Contractant: AT

1. Procédé de préparation des composés de formule 1

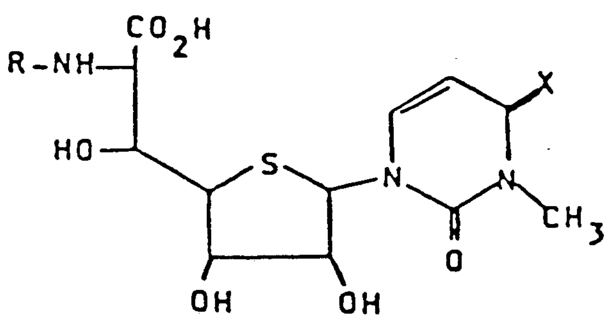

(1)

dans laquelle

X représente 0 ou N-CO-$NH_2$, et

R représente le radical d'un aminoacide (autre que la sérine), un radical d'oligopeptide (autres que HyoHyoHyoSer et ses dérivés sur le groupe amino libre) et un radical du groupe HyoHyoSer, HyoHyoHyoAla, HyoHyoHyoThr, Hyo, HyoHyo ou HyoHyoHyo, Hyo représentant la $N^5$-acétyl-$N^5$-hydroxy-L-ornithine,

caractérisé en ce que l'on soumet un composé de formule 2

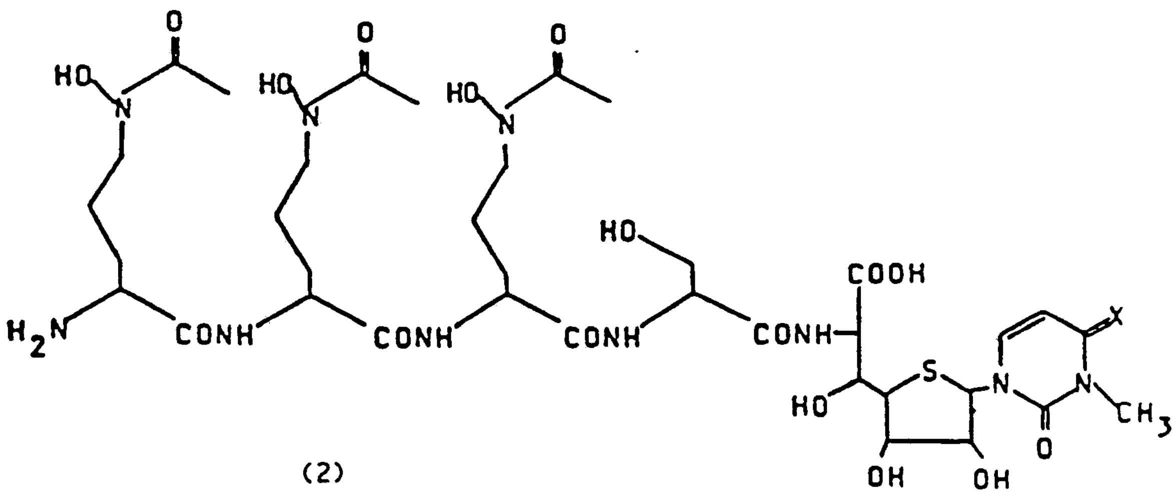

(2)

dans laquelle X représente 0 ou $N-CO-NH_2$,
à scission à l'aide d'une enzyme appropriée en les composés 3 et 4

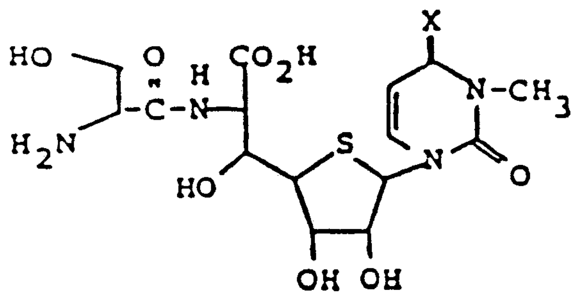

(3)

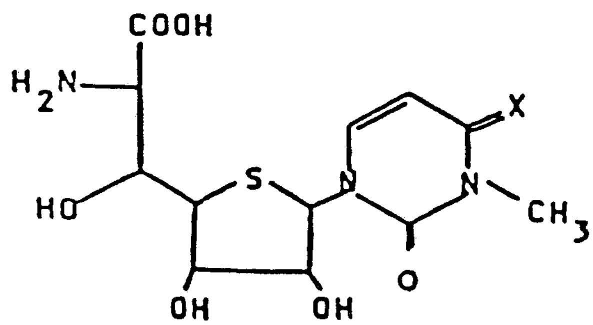

(4)

dans lesquelles X représente 0 ou $N-CO-NH_2$,
on couple les composés 3 et 4 par les oligopeptides ou aminoacides bloqués correspondants au radical R puis on débloque.

2. Procédé selon la revendication 1, caractérisé en ce que, pour la scission de (2) en (3), on utilise des peptides-hydrolases des groupes EC 3.4.11 (α-amino-acyl-peptide-hydrolases) et EC 3.4.21-23 (protéases).

3. Procédé selon la revendication 2, caractérisé en ce que, pour la scission de (2) en (3), on utilise de l'α-chymotrypsine, de la substilisine, de l'amino-acylase I, de la β-lactamase d'E. Coli T7, de la protéinase K ou de la pronase K.

4. Procédé selon la revendication 1, caractérisé en ce que, pour la scission de (2) en (4), on utilise une leucinaminopeptidase microsomique.

5. Procédé selon la revendication 4, caractérisé en ce que la scission est effectuée en milieu aqueux à un pH de 6,6 à 7,8 et à une température de réaction de 20 à 40° C.

6. Procédé selon la revendication 1, caractérisé en ce que les aminoacides ou peptides correspondant au radical R qui sont mis en oeuvre sont bloqués à l'azote.

7. Procédé selon la revendication 6, caractérisé en ce que les aminoacides et peptides correspondant au

radical R sont bloqués par des groupes tert-butyloxycarbonyle ou benzyloxycarbonyle.

8. Procédé selon la revendication 1, caractérisé en ce que la liaison du peptide ou oligopeptide avec les composés de formules 3 et 4 est réalisée par le procédé à l'anhydride mixte.

9. Procédé selon la revendication 1, caractérisé en ce que le couplage avec les composés (3) et (4) est effectué dans un milieu hydro-organique, en particulier dans des mélanges eau/tétrahydrofuranne.

**Revendications** pour les Etats Contractants: BE, CH, DE, FR, GB, IT, LI, NL, SE

1. Composés de formule générale 1

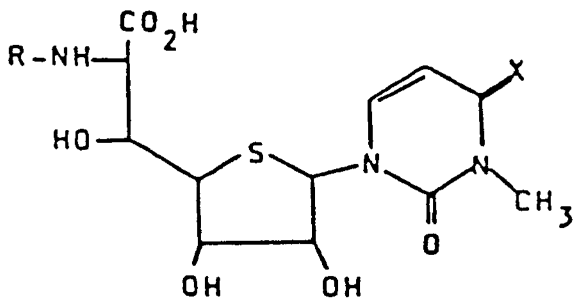

(1)

dans laquelle

X représente 0 ou N-CO-$NH_2$- et

R représente le radical d'un aminoacide (autre que la sérine), un radical d'oligopeptide (autre que HyoHyoHyoSer et ses dérivés sur le groupe amino libre) et un radical du groupe HyoHyoSer, HyoHyoHyoAla, HyoHyoHyoThr, Hyo, HyoHyo ou HyoHyoHyo, Hyo représentant la $N^5$-acétyl-$N^5$-hydroxy-L-ornithine.

2. Composés selon la revendication 1, caractérisés en ce que

R représente la glycine, l'alanine, la valine, la leucine, l'isoleucine, la phénylalanine, la tyrosine, la proline, l'hydroxyproline, la thréonine, la cystéine, la cystine, la méthionine, le tryptophane, l'acide aspartique, l'asparagine, l'acide glutamique, la glutamine, l'acide hydroxyglutamique, l'arginine, la lysine, l'histidine ou un radical d'oligopeptide consistant en ces aminoacides.

3. Composés selon la revendication 1 ou 2, dans lesquels R représente l'alanine ou la thréonine.

4. Composé de formule 4

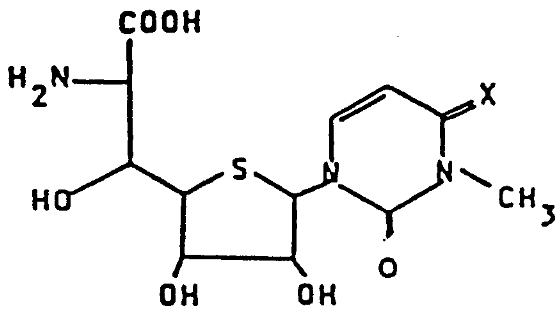

(4)

dans laquelle X représente l'oxygène ou N-CO-$NH_2$.

5. Procédé de préparation des composés selon la revendication 1, caractérisé en ce que l'on soumet des composés de formule 2

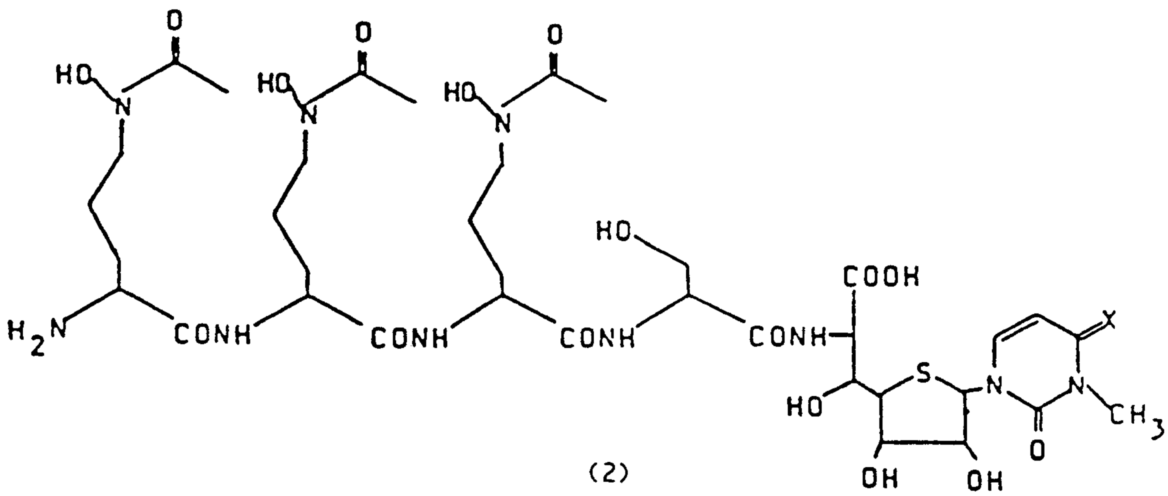

(2)

dans laquelle X = 0 ou $N\text{-}CO\text{-}NH_2$, à scission à l'aide d'enzymes appropriées, en composés de formule 3

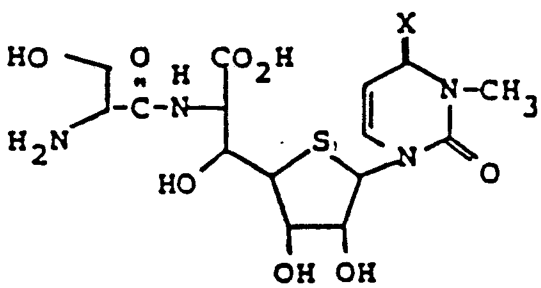

(3)

puis à l'aide de leucinaminopeptidase microsomique, en composés de formule 4

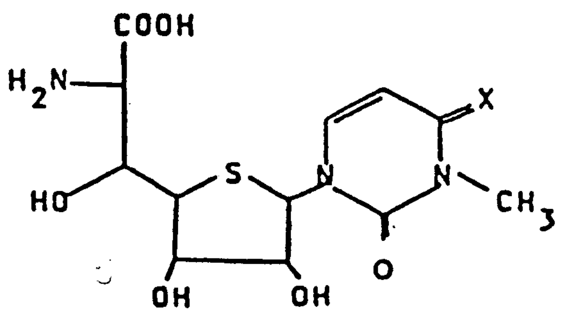

(4)

ou bien on scinde directement les composés de formule 2, à l'aide de leucinaminopeptidase microsomique, en les composés de formule 4, puis on relie les composés de formules 3 et 4 par un aminoacide correspondant au radical R de la revendication 1 ou par un dérivé activé d'un tel acide.

6. Médicament contenant au moins un composé de formule générale 1 selon la revendication 1.

7. Composés de formule générale 1 selon la revendication 1, pour l'utilisation dans un procédé pour le traitement thérapeutique de l'organisme humain ou animal.

8. Utilisation des composés de formule générale 1 selon la revendication 1 pour la préparation de médicaments.